# EUROPÄISCHE PATENTSCHRIFT

(11) **EP 3 164 193 B1**
(45) Veröffentlichungstag und Bekanntmachung des Hinweises auf die Patenterteilung: **27.03.2019**
(21) Anmeldenummer: 15727320.2
(22) Anmeldetag: 18.05.2015
(51) Int. Cl.: A61P 17/14, A61K 8/64, A61Q 5/10, A61Q 5/06

(54) **VERWENDUNG VON SPEZIELLEN OLIGOPEPTIDEN ZUR STIMULIERUNG DES NATÜRLICHEN PIGMENTIERUNGSPROZESSES IN HAUTANHANGSGEBILDEN**
USE OF SPECIFIC OLIGOPEPTIDES FOR STIMULATING THE NATURAL PIGMENTATION PROCESS IN CUTANEOUS APPENDAGES
UTILISATION D'OLIGOPEPTIDES SPÉCIAUX POUR LA STIMULATION DU PROCESSUS NATUREL DE PIGMENTATION DES ANNEXES CUTANÉES

(30) Priorität: 03.07.2014 DE 102014212921
(43) Veröffentlichungstag der Anmeldung: 10.05.2017
(73) Patentinhaber: Henkel AG & Co. KGaA, 40589 Düsseldorf (DE)
(72) Erfinder: GIESEN, Melanie, 47608 Geldern (DE); FUHRMANN, Guido, 41812 Erkelenz (DE)
(86) Internationale Anmeldenummer: PCT/EP2015/060870
(87) Internationale Veröffentlichungsnummer: WO 2016/000867

(56) Entgegenhaltungen:
- WO-A1-2005/116068
- WO-A2-2007/024899
- WO-A2-2009/068688
- DE-A1-102007 033 650
- JP-A- 2009 035 525
- US-A1- 2003 086 893

## Beschreibung

Die vorliegende Erfindung betrifft eine kosmetische nicht-therapeutische Verwendung mindestens eines speziellen Oligopeptids zur Stimulierung des natürlichen Pigmentierungsprozesses in Hautanhangsgebilden, insbesondere in menschlichen Haaren und Haarfollikeln.

Weiterhin betrifft die vorliegende Erfindung eine kosmetische, nicht-therapeutische Verwendung eines kosmetischen Mittels, welches mindestens ein spezielles Oligopeptid enthält, zur Stimulierung des natürlichen Pigmentierungsprozesses in Hautanhangsgebilden, insbesondere in menschlichen Haaren und Haarfollikeln.

Darüber hinaus betrifft die vorliegende Erfindung ein kosmetisches nicht-therapeutisches Verfahren zur Stimulierung des natürlichen Pigmentierungsprozesses von Hautanhangsgebilden, insbesondere der Melanogenese und/oder der Pigmentierung des Haares, zur Verhinderung und/oder der Verringerung der Haarergrauung und/oder zur Repigmentierung von ergrautem Haar, bei welchem ein spezielles Oligopeptid auf Hautanhangsgebilde, insbesondere menschlichen Haare und Haarfollikel, appliziert und nach einer bestimmten Einwirkzeit ausgespült wird.

Schließlich betrifft die vorliegende Erfindung ein kosmetisches nicht-therapeutisches Verfahren zur Stimulierung des natürlichen Pigmentierungsprozesses von Hautanhangsgebilden, insbesondere der Melanogenese und/oder der Pigmentierung des Haares, zur Verhinderung und/oder der Verringerung der Haarergrauung und/oder zur Repigmentierung von ergrautem Haar, bei welchem ein kosmetisches Mittel, enthaltend mindestens ein spezielles Oligopeptid, auf Hautanhangsgebilde, insbesondere menschliche Haare und Haarfollikel, appliziert und nach einer bestimmten Einwirkzeit ausgespült wird.

Haare besitzen neben ihren eigentlichen physiologischen Aufgaben in Form der Wärmeisolierung und des Lichtschutzes eine nicht zu unterschätzende psychosoziale Funktion. Sie sind unter anderem ein Mittel der zwischenmenschlichen Kommunikation und stellen ein Zeichen der eigenen Individualität dar. Veränderungen, wie beispielsweise die Ergrauung, können zu Beeinträchtigungen des Selbstbewusstseins der betroffenen Personen führen. Es besteht daher bei Verbrauchern der Wunsch, ergraute Haare zu kolorieren.

Hierzu können die Haare beispielsweise mit Hilfe von chemischen Colorationen behandelt werden, welche aufgrund ihrer chemischen Aggressivität eine mangelnde Verträglichkeit (Jucken, Brennen, Stechen) aufweisen können und zu einer Schädigung der Haare bei dauerhafter Anwendung führen können. Darüber hinaus muss bei Verwendung derartiger Colorationen der Haaransatz regelmäßig nachgefärbt werden, um eine einheitliche Haarfarbe zu gewährleisten.

Weiterhin sind im Stand der Technik Produkte bekannt, in welchen biologische Produkte als Farbstoffe zur Grauabdeckung eingesetzt werden. Diese Produkte weisen jedoch nicht immer eine zufriedenstellende Leistung auf.

Um die zuvor angeführten Nachteile zu vermeiden, erscheint es daher wünschenswert, den Ergrauungsprozess direkt an der Haarwurzel bzw. in den Haarfollikeln zu beeinflussen um auf diese Weise eine Repigmentierung der menschlichen Haare bzw. eine Verminderung oder sogar eine Beseitigung der Haarergrauung zu erreichen.

Die Pigmentierung im Haarfollikel erfolgt durch die Synthese von Melanin, einem Farbstoff, welcher in den Melanosomen der Melanocyten produziert und gespeichert wird. Melanin ist für die natürliche Haarfarbe verantwortlich, wobei die Haarfarbe durch die Anteile von zwei verschiedenen Arten von Melanin, dem Eu- und Pheomelanin, gesteuert wird. Die Synthese des Melanins, welche auch als Melanogenese bezeichnet wird, wird durch ein definiertes komplexes Set molekularer Signale gesteuert. Bei der Melanogenese erfolgt zunächst die Umsetzung von Tyrosin mit dem Enzym Tyrosinase in L-Dihydroxyphenylalanin (auch als L-DOPA bezeichnet), welches anschließend über mehrere weitere Zwischenstufen zu verschiedenen Melaninpigmenten umgesetzt wird. Die Beeinflussung bzw. Störung der Melanogenese in ergrauten Haarfollikeln kann beispielsweise durch die Modifizierung der Funktion der molekularen Signale entstehen. Eine Folgeerscheinung dieser Modifizierung ist die Verminderung der Melaninsynthese, welche zum Ergrauen des Follikels führt. Zu dem komplexen Set molekularer Signale, welche die Melanogenese beeinflussen, gehören unter anderem die Expression von MCR1 (Melanocortinrezeptor 1), gp100 sowie ckit. MCR1 und ckit sind Rezeptoren, welche Schlüsselsignale der Melanogenese durch die Bindung ihrer Liganden alphamelanocyte stimulating hormone und stem cell factor ins Zellinnere weiterleiten. Gp100 ist ein Protein der Melanosomenmembran und reguliert darüber hinaus weitere melanogneserelevante Proteine. Da diese Parameter von essentieller Bedeutung bei der Haarfollikelpigmentierung sind, kann durch die Beeinflussung dieser Signale die Melaninsynthese in den Haarfollikelzellen aufrechterhalten bzw. sogar reaktiviert werden. Durch den Einsatz geeigneter Wirkstoffformulierungen ist es daher möglich, die Pigmentierung und damit die Jugendlichkeit der Haare zu erhalten.

Aus dem Stand der Technik sind bereits Wirkstoffe bekannt, welche zu einer Stimulierung der Pigmentierung von Haut und Haaren führen. So beschreibt die Offenlegungsschrift WO 91/07945 A1 die Verwendung von Xanthinen als Wirkstoffe. Darüber hinaus ist aus der Offenlegungsschrift DE 10 2009 044 964 A1 auch der Einsatz von Carnitin oder dessen Derivaten in Kombination mit Purin oder dessen Derivaten zur Pigmentierung von menschlichen Haaren bekannt

Die DE 10 2007 033650 A1 offenbart die kosmetische Verwendung mindestens eines Oligopeptids, welches mindestens eine Aminosäuresequenz der Formel

(H)NH-(A)a-(B)b·Glu-Glu-Glu-(C)c-(D)d-(E)e-(Fx·CO-(OH)

umfasst, worin A, B, C, D, E und F, jeweils unabhängig voneinander, für die Aminosäuren Leu, Tyr, lle, Arg, Val, Ser, Thr und Asp stehen,a, b, c, d, e und f, jeweils unabhängig voneinander, für die ganzen Zahlen 0 oder 1 stehen, die Amino-Gruppe nicht protoniert und/oder protoniert vorliegt und die Carboxy-Gruppen nicht deprotoniert und/oder deprotoniert vorliegen zur Pflege von Haaren. Auch die Verzögerung des Alterungsprozesses von keratinischen Fasern ist genannt, jedoch keine Verwendung der Peptide zur Stimulierung des natürlichen Pigmentierungsprozesses in Haar oder Haarfollikeln.

Die WO 2009/068688 A2 offenbart die Verwendung von Peptiden zur Stimulierung des natürlichen Pigmentierungsprozesses in Haar oder Haarfollikeln. Auch die WO 2005/116068 A1 offenbart die Verwendung von Peptiden zur Stimulierung des natürlichen Pigmentierungsprozesses in Haar oder Haarfollikeln.

Auch die US 2003/086893 A1, die WO 2007/024899 A2 und die JP 2009 035525 A offenbaren Peptide. Eine Verwendung zur Stimulation der Pigmentbildung ist in diesen Dokumenten jedoch nicht genannt.

Es besteht im Stand der Technik jedoch weiterhin ein Bedarf für die Bereitstellung hochpotenter Wirkstoffe bzw. Wirkstoffkombinationen, welche die Melanogenese im Haarfollikel beeinflussen und zu einer Repigmentierung von menschlichen Haaren führen.

Der vorliegenden Anmeldung lag die Aufgabe zugrunde, Wirkstoffe bereitzustellen, welche die Nachteile des Standes der Technik vermeiden bzw. vermindern und welche zu einer signifikanten Beeinflussung des natürlichen Pigmentierungsprozesses, insbesondere im Haar bzw. im Haarfollikel, führen. Weiterhin sollen sich diese Wirkstoffe problemlos in kosmetische Zusammensetzungen einarbeiten lassen, ohne zu Stabilitätsproblemen oder Inkompatibilitäten mit anderen Inhaltsstoffen zu führen.

Es wurde nun überraschend gefunden, dass die Verwendung von mindestens einem speziellen Oligopeptid zu einer Stimulierung des natürlichen Pigmentierungsprozesses in Hautanhangsgebilden, wie menschlichen Haaren und Haarfollikeln, insbesondere zu einer signifikant gesteigerten Synthese von Melanin in den Hautanhangsgebilden, führt, und auf diese Weise eine hervorragende Repigmentierung der menschlichen Haare bzw. eine Verhinderung der Ergrauung von menschlichen Haaren erreicht werden kann.

Gegenstand der vorliegenden Erfindung ist daher eine kosmetische, nicht-therapeutische Verwendung mindestens eines Oligopeptids a), welches die Aminosäuresequenz

(H)NH-Leu-Tyr-Glu-Glu-Glu-Ile-Arg-Val-Leu-CO(OH)

aufweist, und mindestens eines Oligopeptids b), welches die Aminosäuresequenz

(H)NH-Tyr-Glu-Glu-Glu-Ile-Arg-Val-Leu-CO(OH)

aufweist, wobei die Oligopeptide in einer Gesamtmenge von 0,001 bis 3 Gew.-%, bezogen auf das Gesamtgewicht einer Zusammensetzung, enthaltend die Oligopeptide, gegebenenfalls einen Träger sowie gegebenenfalls weitere Inhaltsstoffe, verwendet werden,
zur Stimulierung des natürlichen Pigmentierungsprozesses in Haaren und Haarfollikeln.

Die Verwendung der zuvor genannten speziellen Oligopeptide führt zu einer Stimulierung des natürlichen Pigmentierungsprozesses in Hautanhangsgebilden, insbesondere in menschlichen Haaren und Haarfollikeln, welche sich in einer signifikant gesteigerten Synthese von Melanin in den Hautanhangsgebilden manifestiert. Zudem lassen sich diese speziellen Oligopeptide ohne Probleme in kosmetische Mittel zur Repigmentierung menschlicher Haare einarbeiten und führen nicht zu Inkompatibilitäten bzw. Unverträglichkeiten mit den in diesen Mitteln eingesetzten Inhaltsstoffen, so dass bei Verwendung der zuvor beschriebenen speziellen Oligopeptiden eine hohe Lagerstabilität der kosmetischen Mittel gewährleistet wird.

Unter dem Begriff "Oligopeptid" im Rahmen der vorliegenden Erfindung werden Kondensationsprodukte von Aminosäuren verstanden, welche durch Peptid-Bindungen Säureamid-artig verknüpft sind und 3 bis 25 Aminosäuren umfassen.

Weiterhin wird unter dem Begriff "Stimulierung des natürlichen Pigmentierungsprozesses" im Rahmen der vorliegenden Erfindung die positive Beeinflussung der durch den Alterungsprozess abnehmenden natürlichen Farbgebung/Färbung/Pigmentierung von Hautanhangsgebilden, insbesondere die positive Beeinflussung des durch den Alterungsprozess abnehmenden natürlichen, d.h. biologischen, Pigmentierungsprozesses in Hautanhangsgebilden verstanden. Hierunter fällt jedoch nicht die positive Beeinflussung der durch Krankheit bedingten abnehmenden natürlichen Farbgebung/Färbung/Pigmentierung von Hautanhangsgebilden.

Im Rahmen der vorliegenden Anmeldungen werden die in dem erfindungsgemäß verwendeten Oligopeptid enthaltenen Aminosäuren mit dem sogenannten 3-Letter-Code abgekürzt. Im Folgenden sind die im Rahmen der vorliegenden Anmeldung verwendeten Aminosäuren sowie deren Abkürzungen im 3-Letter-Code aufgeführt:
Glu = Glutaminsäure; Leu = Leucin; Tyr = Tyrosin; Ile = Isoleucin; Arg = Arginin; Val = Valin; Ser = Serin; Thr = Threonin; Asp = Asparaginsäure.

In obiger Formel (I) sowie in allen nachstehenden Formeln bedeutet das eingeklammerte Wasserstoffatom der Aminogruppe ebenso wie die eingeklammerte Hydroxygruppe der Säurefunktion, dass die betreffenden Gruppen als solche vorhanden sein können (dann handelt es sich um ein Oligopeptid mit der betreffenden Anzahl an Aminosäuren wie beispielsweise in der obigen Formel (I)) dargestellt. In diesem Fall können die Amino-Gruppe frei oder protoniert und die Carboxy-Gruppen frei oder deprotoniert vorliegen. Es ist im Rahmen der vorliegenden Erfindung jedoch auch möglich, dass die Aminosäuresequenz in einem Oligopeptid vorliegt, welches noch weitere Aminosäuren umfasst. In diesem Fall sind der/die eingeklammerte(n) Bestandteil(e) der obigen Formel (I) sowie aller nachstehenden Formeln durch den oder die weiteren Aminosäurereste ersetzt, deren Amino-Gruppen frei oder protoniert und deren Carboxy-Gruppen frei oder deprotoniert vorliegen können.

Gemäß einer bevorzugten Ausführungsform der vorliegenden Erfindung wird durch die erfindungsgemäße Verwendung der natürliche Pigmentierungsprozess des Haares stimuliert. Unter der Bezeichnung "Haare" sind im Rahmen der vorliegenden Erfindung sowohl keratinische Fasern als solche als auch die Haarfollikel, insbesondere menschliche keratinische Fasern und Haarfollikel, zu verstehen. Bevorzugt bezeichnet dieser Begriff menschliche Kopf- und Barthaare sowie deren Haarfollikel.

Im Rahmen der vorliegenden Erfindung ist es besonders bevorzugt, wenn durch die erfindungsgemäße Verwendung mindestens ein Teilschritt des natürlichen Pigmentierungsprozesses stimuliert wird. Diese Stimulation betrifft insbesondere die Regulation solcher molekularer Signale, welche den biologischen bzw. natürlichen Pigmentierungsprozess beeinflussen. Bevorzugt ist die Regulation des biologischen bzw. natürlichen Pigmentierungsprozesses durch Genregulation, wie beispielsweise die Regulation auf Expressionsebene, und/oder durch Enzymregulation, wie beispielsweise die Regulation auf Aktivitätsebene, und/oder durch die Regulation auf Hormonebene. Die Stimulierung eines Teilschritts des natürlichen Pigmentierungsprozesses kann im Rahmen dieser Ausführungsform beispielsweise durch die Genregulation oder die Enzymregulation der Tyrosinase erfolgen.

Weiterhin ist es im Rahmen einer Ausführungsform der vorliegenden Erfindung bevorzugt, dass durch die erfindungsgemäße Verwendung die Pigmentierung des Haares stimuliert und/oder verbessert wird. Unter dem Begriff der "Stimulierung bzw. Verbesserung" wird im Rahmen der vorliegenden Erfindung insbesondere die Verbesserung, Erhöhung und/oder Stimulierung der Transports der Melanosomen in die den Haarfollikel umgebende Keratinozyten sowie weiterhin die mit dem Auge oder entsprechend geeigneten Messmethoden wahrnehmbare Pigmentierung des einzelnen Haares, einer Auswahl von Haaren, insbesondere einem Areal behaarter Haut, insbesondere der Kopfhaut, oder des gesamten Kopf- und/oder Barthaares, verstanden.

Gemäß einer besonders bevorzugten Ausführungsform der vorliegenden Erfindung wird durch die erfindungsgemäße Verwendung die Melanogenese, insbesondere im Haarfollikel, stimuliert. Unter Stimulierung der Melanogenese, bevorzugt der Melanogenese im Haarfollikel, ist erfindungsgemäß besonders bevorzugt die Stimulierung, Erhöhung, Anregung bzw. Verbesserung der Melaninsynthese in den Melanocyten (bevorzugt der Melanocyten im Haarfollikel) zu verstehen. Dies wird beispielsweise durch eine Erhöhung der Genexpression von Signalmolekülen wie MCR1 (Melanocortinrezeptor 1), gp100 sowie ckit erreicht. Insbesondere wird die Melanogenese im Haar bzw. Haarfollikel der behaarten Kopfhaut und/oder des Bartes, insbesondere der behaarten Kopfhaut und/oder des Bartes des Menschen, stimuliert.

Im Rahmen der vorliegenden Erfindung ist es bevorzugt, wenn durch die erfindungsgemäße Verwendung die Haarergrauung verhindert und/oder verringert wird. Unter dem Begriff der "Haarergrauung" ist im Sinne der vorliegenden Erfindung die optisch durch die Mischung weißer und pigmentierter Haare wahrnehmbare Haarergrauung, die Pigmentverdünnung in einem einzelnen Haar, sowie die Ergrauung eines einzelnen Haares, durch den natürlichen Alterungsprozess zu verstehen. Hierunter fällt jedoch nicht die krankhafte oder krankheitsbedingte Ergrauung der Haare.

Eine Verhinderung der Haarergrauung erfolgt insbesondere bei noch nicht ergrauten Haaren. Dahingegen kann eine Verringerung der Haarergrauung sowohl bei bereits ergrautem als auch bei noch nicht ergrauten Haaren stattfinden. Bei bereits ergrauten Haaren werden Haarfollikel, in welchen die Melanogenese nicht, nicht mehr oder nicht vollständig funktioniert bzw. gestört oder reduziert ist, wieder zur Melanogenese angeregt/stimuliert, während in nicht ergrauten Haaren/Haarfollikeln eine Störung, Reduktion bzw. Herunterregulation der Melanogenese verhindert bzw. vermindert wird.

Gemäß einer weiteren bevorzugten Ausführungsform der vorliegenden Erfindung kann es vorgesehen sein, dass durch die erfindungsgemäße Verwendung das ergraute Haar repigmentiert wird.

Im Rahmen der vorliegenden Erfindung ist es bevorzugt, wenn die Verwendung topisch erfolgt. Unter einer topischen Anwendung wird erfindungsgemäß das Auftragen auf die Hautanhangsgebilde, insbesondere die Kopfhaut sowie die Gesichtshaut, verstanden.

Gemäß einer besonders bevorzugten Ausführungsform der vorliegenden Erfindung weist das mindestens eine Oligopeptid einen Tyrosingehalt von weniger als 6%, vorzugsweise von weniger als 5 %, bevorzugt von weniger als 4 %, insbesondere von weniger als 3 %, auf. Die Verwendung von mindestens einem Oligopeptid führt trotz des geringen Anteils an Tyrosin, welches als Ausgangsstoff in der Melaninsynthese dient, zu einer hervorragenden Stimulierung des natürlichen Pigmentierungsprozesses in Form der Erhöhung der Melaninsynthese und damit zu einer besonders effektiven Repigmentierung von ergrauten Haaren. Die Bestimmung des Tyrosingehalts der erfindungsgemäß verwendeten Oligopeptide kann mithilfe verschiedener Analyseverfahren, wie dem Edman-Abbau (P. Edman, A method for the determination of amino acid sequence in peptides.; Arch. Biochem.. 22, 1949, Seite 475) oder mittels HPLC-Verfahren (Cohen, et al, "Compositional Protein Analysis using 6-Aminoquinolyl-N-Hydroxasuccinimyl Carbamate, a Novel Derivatization Reagent", Techniques in Protein Chemistry IV, 1993, Seiten 289 bis 298) bestimmt werden.

Im Rahmen der vorliegenden Erfindung werden zwei voneinander verschiedene Oligopeptide verwendet, welche sich in mindestens einer Aminosäure voneinander unterscheiden.

Im Rahmen der vorliegenden Erfindung weist das Oligopeptid a) die Aminosäuresequenz (H)NH-Leu-Tyr-Glu-Glu-Glu-Ile-Arg-Val-Leu-CO(OH) und das Oligopeptid b) die Aminosäuresequenz (H)NH-Tyr-Glu-Glu-Glu-Ile-Arg-Val-Leu-CO(OH) auf. Die erfindungsgemäße Verwendung von zwei voneinander verschiedenen Oligopeptiden a) und b) führt zu einer hervorragenden Stimulierung des natürlichen Pigmentierungsprozesses in Form der Melaninsynthese und somit zu einer signifikanten Steigerung des Melaningehalts in den Haarfollikeln. Durch die Erhöhung des Melaningehalts kann die Ergrauung der Haare vermieden bzw. vermindert oder sogar beseitigt werden.

Die im Rahmen der vorliegenden Erfindung verwendeten Oligopeptide, welche den vorstehend genannten Bedingungen genügen, können vorteilhafterweise aus keratinischen Materialien gewonnen werden. Es ist erfindungsgemäß bevorzugt, dass diese Oligopeptide in hohen Anteilen, bezogen auf den gesamten keratinischen Peptidgehalt vorliegen. Ganz besonders bevorzugt ist es daher, dass ein möglichst hoher Anteil aller erfindungsgemäß verwendeten keratinischen Oligopeptide den vorstehend genannten Bedingungen genügt.

Erfindungsgemäß bevorzugt werden die Oligopeptide a) und b) in einer Gesamtmenge von 0,001 bis 3 Gew.-%, vorzugsweise von 0,002 bis 2,8 Gew.-%, bevorzugt von 0,005 bis 2,5 Gew.-%, weiter bevorzugt von 0,008 bis 2,3 Gew.-%, insbesondere von 0,01 bis 2,0 Gew.-%, bezogen auf das Gesamtgewicht einer Zusammensetzung, enthaltend die Oligopeptide a) und b), gegebenenfalls einen Träger sowie gegebenenfalls weitere Inhaltsstoffe, verwendet. Die Verwendung der Oligopeptide a) und b) in den zuvor genannten Mengen resultiert in einer signifikanten Erhöhung der Melaninsynthese durch eine Stimulierung des natürlichen Pigmentierungsprozesses. Darüber hinaus kommt es bei Verwendung dieser Mengen der Oligopeptide a) und b) nicht zu negativen Wechselwirkungen mit gegebenenfalls weiteren eingesetzten Inhaltsstoffen, so dass die Zusammensetzungen trotz Einsatz des mindestens einen Oligopeptids eine hohe Stabilität aufweisen.

Die zuvor genannte Gesamtmenge der Oligopeptide a) und b) bezieht sich hierbei auf eine Zusammensetzung, welche neben den Oligopeptiden a) und b) bevorzugt mindestens einen kosmetisch verträglichen Träger sowie gegebenenfalls weitere in kosmetischen Zusammensetzungen übliche Inhaltsstoffen aufweist.

Ein weiterer Gegenstand der vorliegenden Erfindung ist eine kosmetische, nicht-therapeutische Verwendung eines kosmetischen Mittels zur Stimulierung des natürlichen Pigmentierungsprozesses in Haaren und Haarfollikeln, wobei das kosmetische Mittel in einem kosmetisch verträglichen Träger mindestens ein Oligopeptid a), welches die Aminosäuresequenz (H)NH-Leu-Tyr-Glu-Glu-Glu-Ile-Arg-Val-Leu-CO(OH) aufweist, und mindestens eines Oligopeptids b), welches die Aminosäuresequenz (H)NH-Tyr-Glu-Glu-Glu-Ile-Arg-Val-Leu-CO(OH) aufweist, in einer Gesamtmenge von 0,001 bis 3 Gew.-%, bezogen auf das Gesamtgewicht des Mittels, enthält.

Der Einsatz von kosmetischen Mittel mit speziellen Oligopeptiden führt zu einer Stimulierung des natürlichen Pigmentierungsprozesses in Hautanhangsgebilden, insbesondere in menschlichen Haaren bzw. Haarfollikeln, in Form einer gesteigerten Synthese des natürlichen Haarfarbstoffs Melanin. Auf diese Weise wird eine effektive Verminderung der Haarergrauung bzw. sogar eine Vermeidung der Haarergrauung gewährleistet.

Die erfindungsgemäß verwendeten kosmetischen Mittel liegen bevorzugt in Form von Haarbehandlungsmitteln vor. Haarbehandlungsmittel im Sinne der vorliegenden Erfindung sind beispielsweise Haarfärbemittel, Blondiermittel, Haarshampoos, Haarkonditionierer, konditionierende Shampoos, Haarsprays, Haarspülungen, Haarkuren, Haarpackungen, Haar-Tonics, Dauerwell-Fixierlösungen, Haarfärbeshampoos, Haarfärbemittel, Haarfestiger, Haarlegemittel, Haarstyling-Zubereitungen, Fönwell-Lotionen, Schaumfestiger, Haargele, Haarwachse oder deren Kombinationen. Besonders bevorzugte Haarbehandlungsmittel sind dadurch gekennzeichnet sind, dass sie als Shampoo, Haar-Tonics, Haarkur, Haarspülung, Haarschaum, Haarfestiger, Haarspray, Haargel und/oder Haarfärbemitte konfektioniert sind. Im Hinblick auf die Tatsache, dass Verbraucher oft die Anwendung mehrerer unterschiedlicher Mittel und/oder mehrere Anwendungsschritte scheuen, ist die erfindungsgemäße Verwendung solcher kosmetischer Mittel bevorzugt, welche der Verbraucher ohnehin anwendet. Bevorzugt verwendete kosmetische Mittel sind daher Shampoos, Konditioniermittel oder Haar-Tonics.

Die erfindungsgemäß verwendeten Mittel enthalten einen kosmetischen Träger. Erfindungsgemäß bevorzugt ist der kosmetische Träger wässrig, alkoholisch oder wässrig-alkoholisch.

Ein wässriger Träger enthält im Sinne der Erfindung mindestens 30 Gew.-%, insbesondere mindestens 50 Gew.-% Wasser, bezogen auf das Gesamtgewicht des kosmetischen Mittels.

Unter wässrig-alkoholischen Trägern sind im Sinne der vorliegenden Erfindung wasserhaltige Zusammensetzungen, enthaltend einen C₁-C₄-Alkohol in einer Gesamtmenge von 3 bis 90 Gew.-%, bezogen auf das Gesamtgewicht des kosmetischen Mittels, insbesondere Ethanol bzw. Isopropanol, zu verstehen.

Die erfindungsgemäßen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel, wobei das Lösungsmittel in einer Gesamtmenge von 0,1 bis 30 Gew.-%, vorzugsweise von 1 bis 20 Gew.-%, insbesondere von 2 bis 10 Gew.-%, bezogen auf das Gesamtgewicht des kosmetischen Mittels, enthalten ist.

Die erfindungsgemäß verwendeten kosmetischen Mittel können zusätzlich mindestens einen konditionierenden Stoff, ausgewählt aus der Gruppe von kationischen Polymeren, katonischen Tensiden, Siliconen, Fettstoffen sowie deren Mischungen, enthalten. Unter dem Begriff "konditionierende Stoffe" sind erfindungsgemäß solche Substanzen zu verstehen, welche auf keratinische Materialien, insbesondere auf die Haare, aufziehen und die physikalischen und sensorischen Eigenschaften sowohl der Haare als auch des Produktes als solchem verbessern. Konditionierungsmittel glätten die oberste Schicht der Haare und machen sie weich und geschmeidig.

Die im Rahmen der vorliegenden Erfindung als konditionierende Stoffe einsetzbaren kationischen Polymere weisen in der Haupt- und/oder Seitenkette eine Gruppe auf, welche "temporär" oder "permanent" kationisch sein kann. Als "permanent kationisch" werden erfindungsgemäß solche Polymere bezeichnet, welche unabhängig vom pH-Wert des Mittels eine kationische Gruppe aufweisen. Dies sind in der Regel Polymere, welche ein quartäres Stickstoffatom, beispielsweise in Form einer Ammoniumgruppe, enthalten. Bevorzugte kationische Gruppen sind quartäre Ammoniumgruppen. Insbesondere solche Polymere, bei welchen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden ist, haben sich als besonders geeignet erwiesen.

Bevorzugte kationische Polymere sind ausgewählt aus der Gruppe von Poly(methacryloyloxyethyltrimethylammoniumchlorid) (INCI: Polyquaternium-37), quaternisierten Cellulose-Derivaten (INCI: Polyquaternium 10), kationischen Alkylpolyglycosiden, kationisertem Honig, kationischen Guar-Derivaten, polymeren Dimethyldiallylammoniumsalzen und deren Copolymeren mit Estern und Amiden von Acrylsäure und Methacrylsäure, Copolymeren des Vinylpyrrolidons mit quaternierten Derivaten des Dialkylaminoalkylacrylats und -methacrylats, Vinylpyrrolidon-Vinylimidazoliummethochlorid-Copolymeren, quaterniertem Polyvinylalkohol, Polyquaternium 2, Polyquaternium-7, Polyquaternium 17, Polyquaternium 18, Polyquaternium 24, Polyquaternium 27 sowie deren Mischungen.

Im Rahmen der vorliegenden Erfindung kann es bevorzugt sein, wenn das/die kationische(n) Polymer(e) in einer Gesamtmenge von 0,05 bis 7,5 Gew.-%, vorzugsweise von 0,1 bis 5 Gew.-%, bevorzugt von 0,2 bis 3,5 Gew.-%, insbesondere von 0,25 bis 2,5 Gew.-%, bezogen auf das Gesamtgewicht des erfindungsgemäß verwendeten kosmetischen Mittels, enthalten sind.

Als konditionierende Stoffe eignen sich im Rahmen der vorliegenden Erfindung ebenfalls kationische Tenside aus der Gruppe der quartären Ammoniumverbindungen und/oder der Esterquats und/oder der Amidoamine.

Bevorzugt verwendete kationische Tenside sind ausgewählt aus der Gruppe von Alkyltrimethylammoniumchloriden mit vorzugsweise 10 bis 18 Kohlenstoffatomen im Alkylrest, Dialkyldimethylammoniumchloride mit vorzugsweise 10 bis 18 Kohlenstoffatomen im Alkylrest, Trialkylmethylammoniumchloride mit vorzugsweise 10 bis 18 Kohlenstoffatomen im Alkylrest, Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid, Tricetylmethylammoniumchlorid, Quaternium-27, Quaternium-83, N-Methyl-N(2-hydroxyethyl)-N,N-(ditalgacyloxyethyl)ammonium-methosulfat, N-Methyl-N(2-hydroxyethyl)-N,N-(distearoyloxyethyl)ammonium-methosulfat, N,N-Dimethyl-N,N-distearoyloxyethyl-ammoniumchlorid, N,N-Di-(2-hydroxyethyl)-N,N-(fettsäureesterethyl)-ammoniumchlorid sowie deren Mischungen.

Das/die kationische(n) Tensid(e) sind in den erfindungsgemäß verwendeten kosmetischen Mitteln bevorzugt in einer Gesamtmenge von 0,5 bis 50 Gew.-%, vorzugsweise von 1 bis 40 Gew.-%, besonders bevorzugt von 1,5 bis 30 Gew.-%, insbesondere von 2 bis 20 Gew.-%, bezogen auf das Gesamtgewicht des kosmetischen Mittels, enthalten.

Weiterhin kann es im Rahmen der vorliegenden Erfindung bevorzugt sein, wenn die erfindungsgemäß verwendeten kosmetischen Mittel als konditionierenden Stoff mindestens ein Silicon enthalten, welches ausgewählt ist aus der Gruppe von
(i) Polyalkylsiloxanen, Polyarylsiloxanen, Polyalkylarylsiloxanen, welche flüchtig oder nicht flüchtig, geradkettig, verzweigt oder cyclisch, vernetzt oder nicht vernetzt sind;
(ii) Polysiloxanen, welche in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan-Grundgerüst, auf das nicht siliconhaltige, organische Monomere gepfropft wurden, welche eine Polysiloxan-Hauptkette aufweisen, auf die in der Kette sowie gegebenenfalls an mindestens einem ihrer Enden mindestens ein organisches Makromer gepfropft wurde, das kein Silicon enthält;
oder deren Gemischen.

Bevorzugte Silicone sind ausgewählt aus Siliconen der Formel Si-I

(CH₃)₃Si-[O-Si(CH₃)₂]ₓ-O-Si(CH₃)₃ (Si-I),

in welcher x für eine Zahl von 0 bis 100, vorzugsweise von 0 bis 50, weiter bevorzugt von 0 bis 20, insbesondere 0 bis 10, steht.

Diese Silicone werden nach der INCI-Nomenklatur als DIMETHICONE bezeichnet.

Bevorzugte Silicone weisen bei 20°C Viskositäten von 0,2 bis 2 mm²s⁻¹ auf, wobei Silicone mit Viskositäten von 0,5 bis 1 mm²s⁻¹ besonders bevorzugt sind.

Besonders bevorzugte erfindungsgemäß verwendete kosmetische Mittel enthalten ein oder mehrere aminofunktionelle Silicone. Unabhängig davon, welche aminofunktionellen Silicone eingesetzt werden, sind erfindungsgemäß verwendete kosmetische Mittel bevorzugt, welche ein aminofunktionelles Silicon enthalten dessen Aminzahl oberhalb von 0,25 meq/g, vorzugsweise oberhalb von 0,3 meq/g, insbesondere oberhalb von 0,4 meq/g, liegt. Bevorzugt verwendete kosmetische Mittel enthalten das/die aminofunktionelle(n) Silicon(e) in einer Gesamtmenge von 0,01 bis 10 Gew.-%, vorzugsweise 0,1 bis 8 Gew.-%, bevorzugt 0,25 bis 7,5 Gew.-%, insbesondere 0,5 bis 5 Gew.%, bezogen auf das Gesamtgewicht des kosmetischen Mittels.

Auch der Einsatz der nach der INCI-Nomenklatur als CYCLOMETHICONE bezeichneten cyclischen Dimethicone ist erfindungsgemäß bevorzugt. Insbesondere cyclische Dimethiconen mit der Formel Si-II haben sich im Rahmen der vorliegenden Erfindung als vorteilhaft erwiesen

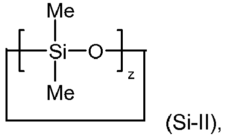

In obiger Formel (Si-II) steht z für eine Zahl von 3 bis 200, vorzugsweise von 3 bis 10, weiter bevorzugt von 3 bis 7, insbesondere für 3, 4, 5 oder 6.

Als besonders geeignet im Rahmen der vorliegenden Erfindung ist die erfindungsgemäße Verwendung von kosmetischen Mitteln, welche das/die Silicon(e) in einer Gesamtmenge von 0,1 bis 10 Gew.-%, vorzugsweise von 0,25 bis 7 Gew.-%, insbesondere von 0,5 bis 5 Gew.-%, bezogen auf das Gesamtgewicht des kosmetischen Mittels, enthalten.

Der Einsatz von Fettstoffen als konditionierende Stoffe ist im Rahmen der vorliegenden Erfindung ebenfalls möglich. Hierbei haben sich insbesondere Fettstoffe aus der Gruppe von pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 Kohlenstoffatomen, z. B. Di-n-octylether und n-Hexyl-n-octylether, Carbonsäuren, besonders linearen und/oder verzweigten, gesättigten und/oder ungesättigten C₈₋₃₀-Carbonsäuren, Alkoholen, besonders gesättigten, ein- oder mehrfach ungesättigten, verzweigten oder unverzweigten Alkoholen mit 4 bis 30 Kohlenstoffatomen, welche mit 1 bis 75, insbesondere 5 bis 20 Ethylenoxid-Einheiten ethoxyliert und/oder mit 3 bis 30, insbesondere 9 bis 14 Propylenoxid-Einheiten propoxyliert sein können, Esterölen, wie Estern von C₆₋₃₀-Carbonsäuren mit C₂₋₃₀-Alkoholen, Hydroxycarbonsäurealkylestern, Dicarbonsäureestern, wie Di-n-butyladipat, sowie Diolestern, wie Ethylenglykoldioleat oder Propylenglykoldi(2-ethylhexanoat), symmetrischen, unsymmetrischen oder cyclischen Estern der Kohlensäure mit Alkoholen, z. B. Glycerincarbonat oder Dicaprylylcarbonat (Cetiol® CC), Mono,- Di- und Tricarbonsäureestern von gesättigten und/oder ungesättigten linearen und/oder verzweigten Carbonsäuren mit Glycerin, welche mit 1 bis 10, insbesondere 7 bis 9 Ethylenoxid-Einheiten ethoxyliert sein können, z. B. PEG-7 Glyceryl Cocoate, bewährt.

Im Rahmen der vorliegenden Erfindung ist es bevorzugt, wenn der/die Fettstoff(e) in den erfindungsgemäß verwendeten kosmetischen Mitteln in einer Gesamtmenge von 0,1 bis 10 Gew.-%, vorzugsweise von 0,25 bis 7 Gew.-%, insbesondere von 0,5 bis 5 Gew.-%, bezogen auf das Gesamtgewicht des kosmetischen Mittels, enthalten sind.

Aus ästhetischen Gründen werden "klare" Produkte von Verbrauchern oft bevorzugt. Bevorzugte erfindungsgemäß verwendete kosmetische Mittel sind daher dadurch gekennzeichnet, dass sie transparent bzw. transluzent sind. Unter transparent oder transluzent wird im Rahmen der vorliegenden Erfindung eine Zusammensetzung verstanden, welche einen NTU-Wert von unter 100 aufweist. Der NTU-Wert (Nephelometric Turbidity Unit, *Nephelometrischer Trübungswert*; NTU) ist eine in der Wasseraufbereitung verwendete Einheit für Trübungsmessungen in Flüssigkeiten. Sie ist die Einheit einer mit einem kalibrierten Nephelometer gemessenen Trübung einer Flüssigkeit.

Bezüglich weiterer bevorzugter Ausführungsformen der erfindungsgemäßen Verwendung des kosmetischen Mittels mit mindestens einem speziellen Oligopeptid gilt mutatis mutandis das zu der erfindungsgemäßen Verwendung von speziellen Oligopeptiden Gesagte.

Darüber hinaus ist ein weiterer Gegenstand der vorliegenden Erfindung ein kosmetisches, nicht-therapeutisches Verfahren zur Stimulierung des natürlichen Pigmentierungsprozesses von Hautanhangsgebilden, insbesondere der Melanogenese und/oder der Pigmentierung des Haares, zur Verhinderung und/oder der Verringerung der Haarergrauung und/oder zur Repigmentierung von ergrautem Haar, welches die nachfolgenden Verfahrensschritte umfasst:
a) Bereitstellen eines Oligopeptids, welches mindestens eine Aminosäuresequenz der Formel (I)

   (H)NH-(A)ₐ-(B)_{b}-Glu-Glu-Glu-(C)_{c}-(D)_{d}-(E)ₑ-(F)_{f}-CO-(OH) (I)

   worin
   A, B, C, D, E und F, jeweils unabhängig voneinander, für die Aminosäuren Leu, Tyr, Ile, Arg, Val, Ser, Thr und Asp stehen,
   a, b, c, d, e und f, jeweils unabhängig voneinander, für die ganzen Zahlen 0 oder 1 stehen, die Amino-Gruppe nicht protoniert und/oder protoniert vorliegt und die Carboxy-Gruppen nicht deprotoniert und/oder deprotoniert vorliegen,
   enthält,
b) Topische Applikation des in Verfahrensschritt a) bereitgestellten mindestens einen Oligopeptids auf Hautanhangsgebilde, insbesondere Haare,
c) Belassen des in Verfahrensschritt b) applizierten mindestens einen Oligopeptids auf den Hautanhangsgebilden, insbesondere Haaren, für einen Zeitraum von 30 Sekunden bis 2 Stunden,
d) gegebenenfalls Ausspülen des in Verfahrensschritt c) auf den Hautanhangsgebilden, insbesondere den Haaren, belassenen kosmetischen Mittels.

Das erfindungsgemäße Verfahren resultiert in einer Stimulierung des natürlichen Pigmentierungsprozesses in Hautanhangsgebilden, insbesondere in menschlichen Haaren bzw. Haarfollikeln, in Form einer gesteigerten Synthese des natürlichen Haarfarbstoffs Melanin. Auf diese Weise wird eine effektive Verminderung der Haarergrauung bzw. sogar eine Vermeidung der Haarergrauung gewährleistet.

Gemäß einer bevorzugten Ausführungsform dieses Gegenstands wird das kosmetische Mittel in Verfahrensschritt c) für einen Zeitraum von 1 Minute bis 1,5 Stunden, vorzugsweise von 2 Minuten bis 1 Stunde, insbesondere von 3 Minuten bis 30 Minuten, auf den Haaren belassen.

Bezüglich weiterer bevorzugter Ausführungsformen des erfindungsgemäßen Verfahrens unter Einsatz der speziellen Oligopeptide gilt mutatis mutandis das zu der erfindungsgemäßen Verwendung von speziellen Oligopeptiden sowie der erfindungsgemäßen Verwendung von kosmetischen Mitteln mit speziellen Oligopeptiden Gesagte.

Schließlich ist ein weiterer Gegenstand der vorliegenden Erfindung ein kosmetisches, nicht-therapeutisches Verfahren zur Stimulierung des natürlichen Pigmentierungsprozesses von Hautanhangsgebilden, insbesondere der Melanogenese und/oder der Pigmentierung des Haares, zur Verhinderung und/oder der Verringerung der Haarergrauung und/oder zur Repigmentierung von ergrautem Haar, umfassend die nachfolgenden Verfahrensschritte:
a) Bereitstellen eines kosmetischen Mittels, umfassend in einem kosmetisch verträglichen Träger mindestens ein Oligopeptid, welches mindestens eine Aminosäuresequenz der Formel (I)

   (H)NH-(A)ₐ-(B)_{b}-Glu-Glu-Glu-(C)_{c}-(D)_{d}-(E)ₑ-(F)_{f}-CO-(OH) (I)

   worin
   A, B, C, D, E und F, jeweils unabhängig voneinander, für die Aminosäuren Leu, Tyr, Ile, Arg, Val, Ser, Thr und Asp stehen,
   a, b, c, d, e und f, jeweils unabhängig voneinander, für die ganzen Zahlen 0 oder 1 stehen, die Amino-Gruppe nicht protoniert und/oder protoniert vorliegt und die Carboxy-Gruppen nicht deprotoniert und/oder deprotoniert vorliegen,
   enthält,
b) Topische Applikation des in Verfahrensschritt a) bereitgestellten kosmetischen Mittels auf Hautanhangsgebilde, insbesondere Haare,
c) Belassen des in Verfahrensschritt b) applizierten kosmetischen Mittels auf den Hautanhangsgebilden, insbesondere Haaren, für einen Zeitraum von 30 Sekunden bis 2 Stunden, und
d) Ausspülen des in Verfahrensschritt c) auf den Hautanhangsgebilden, insbesondere den Haaren, belassenen kosmetischen Mittels.

Das erfindungsgemäße Verfahren resultiert in einer Stimulierung des natürlichen Pigmentierungsprozesses in Hautanhangsgebilden, insbesondere in menschlichen Haaren bzw. Haarfollikeln, in Form einer gesteigerten Synthese des natürlichen Haarfarbstoffs Melanin. Auf diese Weise wird eine effektive Verminderung der Haarergrauung bzw. sogar eine Vermeidung der Haarergrauung gewährleistet.

Gemäß einer bevorzugten Ausführungsform dieses Gegenstands wird das kosmetische Mittel in Verfahrensschritt c) für einen Zeitraum von 1 Minute bis 1,5 Stunden, vorzugsweise von 2 Minuten bis 1 Stunde, insbesondere von 3 Minuten bis 30 Minuten, auf den Haaren belassen.

Bezüglich weiterer bevorzugter Ausführungsformen des erfindungsgemäßen Verfahrens unter Einsatz von kosmetischen Mitteln mit speziellen Oligopeptide gilt mutatis mutandis das zu der erfindungsgemäßen Verwendung von speziellen Oligopeptiden, der erfindungsgemäßen Verwendung von kosmetischen Mitteln mit speziellen Oligopeptiden sowie dem erfindungsgemäßen Verfahren unter Einsatz von speziellen Oligopeptiden Gesagte.

Die folgenden Beispiele erläutern die vorliegende Erfindung, ohne sie jedoch darauf einzuschränken:

### Beispiele:

Zur Untersuchung des Melaningehalts wurden dreidimensionale organotypische Haarfollikelkulturen aus dermalen Papillenzellen, Haarfollikelmelanocyten und Outer Root Sheath Keratinozyten verwendet, welche gemäß dem in der Offenlegungsschrift EP 1 455 854 A1 beschriebenen Verfahren hergestellt wurden. Diese Kulturen wurden über einen Zeitraum von 7 Tagen mit den in Tabelle 1 angegebenen Zusammensetzungen E-I, E-II und E-III behandelt (die Gewichtsangaben in Gew,-% beziehen sich auf das Gesamtgewicht der verwendeten Oligopeptid-Mischung, enthaltend mindestens ein spezielles Oligopeptid sowie Wasser). Als Vergleich (V) dienen unbehandelte Zellkulturen.

| Probe | Oligopeptid-Mischung** [Gew.-%] |
|---|---|
| V | -- |
| E-I* | 0,1 |
| E-II* | 0,5 |
| E-III* | 1,0 |

| | |
|---|---|
| * erfindungsgemäß ** enthaltend - bezogen auf ihr Gesamtgewicht - mindestens 10 Gew.-% einer Mischung aus Oligopeptiden aus 8 Aminosäuren mit der Aminosäuresequenz NH₂-Tyr-Glu-Glu-Glu-Ile-Arg-Val-Leu-COOH und Oligopeptiden aus 9 Aminosäuren mit der Aminosäuresequenz NH₂-Leu-Tyr-Glu-Glu-Glu-Ile-Arg-Val-Leu-COOH und einem Tyrosingehalt von weniger als 3 % | |

Nach 7 Tagen wurden die Haarfollikelkulturen homogenisiert und das Melanin unter Verwendung einer 1M NaOH-Lösung, welche 10 Vol.-% DMSO, bezogen auf das Gesamtvolumen der Extraktionslösung, enthielt, bei 100 °C für 45 Minuten extrahiert. Aliquots dieser Extrakte wurden anschließend in eine 96well-Zellkulturplatte überführt und die Extinktion bzw. die optische Dichte bei einer Wellenlänge von 492 nm mit einem Spektralphotometer Spektramax plus 384 der Firma Molecular Devices (USA) gemessen. Die Auswertung der Ergebnisse erfolgte mit Hilfe der Software Softmax Pro der Firma Molecular Devices (USA).

In Tabelle 2 ist der Melaningehalt aller Haarfollikelkulturen dargestellt. Der Melaningehalt der unbehandelten Haarfollikelkulturen V dient als Referenzwert und entspricht 100 %.

| +Probe | Melaningehalt [%] |
|---|---|
| V | 100 |
| E-I* | 110 |
| E-II* | 114 |
| E-III* | 115 |

| | |
|---|---|
| * erfindungsgemäß | |

Die Verwendung von speziellen Oligopeptiden führt zu einer signifikant erhöhten Synthese von Melanin und somit zu einer signifikanten Stimulation des natürlichen Pigmentierungsprozesses von Haarfollikeln. Durch die Verwendung von speziellen Oligopeptiden kann daher sowohl die Ergrauung der Haare vermieden bzw. vermindert werden als auch eine Repigmentierung, d. h. Verminderung bzw. Beseitigung der Haarergrauung, erreicht werden.

### Sequenzprotokoll:

<210> 1
   <211> 3
   <212> PRT
   <213> Artificial
<220>
   <223> Peptide
<400> 1
   Glu Glu Glu
   1
<210> 2
   <211> 4
   <212> PRT
   <213> Artificial
<220>
   <223> Peptide
<400> 2
   Tyr Glu Glu Glu
   1
<210> 3
   <211> 4
   <212> PRT
   <213> Artificial
<220> <223> Peptide
<400> 3
   Glu Glu Glu Ile
   1
<210> 4
   <211> 6
   <212> PRT
   <213> Artificial
<220>
   <223> Peptide
<400> 4
   Tyr Glu Glu Glu Ile Arg
   1 5
<210> 5
   <211> 7
   <212> PRT
   <213> Artificial
<220>
   <223> Peptide
<400> 5
   Tyr Glu Glu Glu Ile Arg Val
   1 5
<210> 6
   <211> 8
   <212> PRT
   <213> Artificial
<220>
   <223> Peptide
<400> 6
   Tyr Glu Glu Glu Ile Arg Val Leu
   1 5
<210> 7
   <211> 9
   <212> PRT
   <213> Artificial
<220>
   <223> Peptide
<400> 7
   Leu Tyr Glu Glu Glu Ile Arg Val Leu
   1 5

### SEQUENCE LISTING

<110> Henkel AG & Co. KGaA
<120> Verwendung von speziellen Oligopeptiden zur Stimulierung des natürlichen Pigmentierungsprozesses in Hautanhangsgebilden
<130> PT032607 PCT
<140>
   <141>
<150> DE 10 2014 212921.4
   <151> 2014-07-03
<160> 3
<170> PatentIn version 3.5
<210> 1
   <211> 4
   <212> PRT
   <213> Artificial
<220>
   <223> Peptide
<400> 1
   Tyr Glu Glu Glu
   1
<210> 2
   <211> 4
   <212> PRT
   <213> Artificial
<220>
   <223> Peptide
<400> 2
   Glu Glu Glu Ile
   1
<210> 3
   <211> 5
   <212> PRT
   <213> Artificial
<220>
   <223> Peptide
<400> 3
   Tyr Glu Glu Glu Ile
   1               5

## Patentansprüche

1. Kosmetische, nicht-therapeutische Verwendung mindestens eines Oligopeptids a), welches die Aminosäuresequenz (H)NH-Leu-Tyr-Glu-Glu-Glu-Ile-Arg-Val-Leu-CO(OH) aufweist, und mindestens eines Oligopeptids b), welches die Aminosäuresequenz (H)NH-Tyr-Glu-Glu-Glu-Ile-Arg-Val-Leu-CO(OH) aufweist,
wobei die Oligopeptide in einer Gesamtmenge von 0,001 bis 3 Gew.-%, bezogen auf das Gesamtgewicht einer Zusammensetzung, enthaltend die Oligopeptide, gegebenenfalls einen Träger sowie gegebenenfalls weitere Inhaltsstoffe, verwendet werden,
zur Stimulierung des natürlichen Pigmentierungsprozesses in Haaren und Haarfollikeln.

2. Verwendung nach Anspruch 1, **dadurch gekennzeichnet, dass** die Melanogenese, insbesondere im Haarfollikel, stimuliert wird.

3. Verwendung nach einem der Ansprüche 1 oder 2, **dadurch gekennzeichnet, dass** die Oligopeptide in einer Gesamtmenge von 0,002 bis 2,8 Gew.-%, bevorzugt von 0,005 bis 2,5 Gew.-%, weiter bevorzugt von 0,008 bis 2,3 Gew.-%, insbesondere von 0,01 bis 2,0 Gew.-%, bezogen auf das Gesamtgewicht einer Zusammensetzung, enthaltend die Oligopeptide, gegebenenfalls einen Träger sowie gegebenenfalls weitere Inhaltsstoffe, verwendet wird.

4. Kosmetisches, nicht-therapeutisches Verfahren zur Stimulierung des natürlichen Pigmentierungsprozesses von Haaren und Haarfollikeln, insbesondere der Melanogenese und/oder der Pigmentierung des Haares, zur Verhinderung und/oder der Verringerung der Haarergrauung und/oder zur Repigmentierung von ergrautem Haar, welches die nachfolgenden Verfahrensschritte umfasst:
a) Bereitstellen eines Oligopeptids a) welches die Aminosäuresequenz (H)NH-Leu-Tyr-Glu-Glu-Glu-Ile-Arg-Val-Leu-CO(OH) aufweist und eines Oligopeptids b), welches die Aminosäuresequenz (H)NH-Tyr-Glu-Glu-Glu-Ile-Arg-Val-Leu-CO(OH) aufweist in einer Gesamtmenge von 0,001 bis 3 Gew.-%, bezogen auf das Gesamtgewicht einer Zusammensetzung, enthaltend die Oligopeptide, gegebenenfalls einen Träger sowie gegebenenfalls weitere Inhaltsstoffe,
b) Topische Applikation der in Verfahrensschritt a) bereitgestellten Oligopeptide auf Haare und Haarfollikel,
c) Belassen der in Verfahrensschritt b) applizierten Oligopeptide auf den Haaren und Haarfollikeln, für einen Zeitraum von 30 Sekunden bis 2 Stunden,
d) gegebenenfalls Ausspülen des in Verfahrensschritt c) auf den Haaren und Haarfollikeln, belassenen kosmetischen Mittels.

## Claims

1. The cosmetic, non-therapeutic use of at least one oligopeptide a), which has the amino acid sequence (H)NH-Leu-Tyr-Glu-Glu-Glu-Ile-Arg-Val-Leu-CO(OH), and at least one oligopeptide b), which has the amino acid sequence (H)NH-Tyr-Glu-Glu-Glu-Ile-Arg-Val-Leu-CO(OH),
wherein the oligopeptides are used in a total amount of 0.001 to 3 wt.%, based on the total weight of a composition containing the oligopeptides, optionally a carrier and optionally additional ingredients, to stimulate the natural pigmentation process in hair and hair follicles.

2. The use according to claim 1, **characterized in that** melanogenesis, in particular in the hair follicle, is stimulated.

3. The use according to one of claims 1 or 2, **characterized in that** the oligopeptides are used in a total amount of 0.002 to 2.8 wt.%, preferably of 0.005 to 2.5 wt.%, more preferably of 0.008 to 2.3 wt.%, in particular of 0.01 to 2.0 wt.%, based on the total weight of a composition containing the oligopeptides, optionally a carrier and optionally additional ingredients.

4. A cosmetic, non-therapeutic method for stimulating the natural pigmentation process of hair and hair follicles, in particular melanogenesis and/or pigmentation of the hair, for preventing and/or reducing graying of the hair and/or for repigmenting grayed hair, which method comprises the following method steps:
a) providing an oligopeptide a), which has the amino acid sequence (H)NH-Leu-Tyr-Glu-Glu-Glu-Ile-Arg-Val-Leu-CO(OH), and an oligopeptide b), which has the amino acid sequence (H)NH-Tyr-Glu-Glu-Glu-Ile-Arg-Val-Leu-CO(OH), in a total amount of 0.001 to 3 wt.%, based on the total weight of a composition containing the oligopeptides, optionally a carrier and optionally additional ingredients;
b) topically applying the oligopeptides provided in method step a) to hair and hair follicles;
c) leaving the oligopeptides applied in method step b) on the hair and hair follicles for a period of from 30 seconds to 2 hours;
d) optionally rinsing out the cosmetic agent left on the hair and hair follicles in method step c).

## Revendications

1. Utilisation cosmétique et non thérapeutique d'au moins un oligopeptide a) présentant la séquence d'acides aminés (H)NH-Leu-Tyr-Glu-Glu-Glu-Ile-Arg-Val-Leu-CO(OH), et d'au moins un oligopeptide b) présentant la séquence d'acides aminés (H)NH-Tyr-Glu-Glu-Glu-Ile-Arg-Val-Leu-CO(OH), les oligopeptides étant utilisés en une quantité totale située dans la plage allant de 0,001 à 3 % en poids, par rapport au poids total d'une composition contenant les oligopeptides, éventuellement un support et éventuellement d'autres ingrédients, pour la stimulation du processus naturel de pigmentation des cheveux et des follicules capillaires.

2. Utilisation selon la revendication 1, **caractérisée en ce que** la mélanogénèse, en particulier dans le follicule capillaire, est stimulée.

3. Utilisation selon l'une des revendications 1 ou 2, **caractérisée en ce que** les oligopeptides sont utilisés en une quantité totale située dans la plage allant de 0,002 à 2,8 % en poids, de préférence de 0,005 à 2,5 % en poids, de manière davantage préférée de 0,008 à 2,3 % en poids, et en particulier de 0,01 à 2,0 % en poids, par rapport au poids total d'une composition contenant les oligopeptides, éventuellement un support et éventuellement d'autres ingrédients.

4. Procédé cosmétique et non thérapeutique de stimulation du processus naturel de pigmentation des cheveux et des follicules capillaires, en particulier de la mélanogénèse et/ou la pigmentation des cheveux, de prévention et/ou de réduction du blanchissement des cheveux et/ou de repigmentation des cheveux blancs, comprenant les étapes de procédé suivantes :
a) fourniture d'un oligopeptide a) présentant la séquence d'acides aminés (H)NH-Leu-Tyr-Glu-Glu-Glu-Ile-Arg-Val-Leu-CO(OH), et d'au moins un oligopeptide b) présentant la séquence d'acides aminés (H)NH-Tyr-Glu-Glu-Glu-Ile-Arg-Val-Leu-CO(OH) en une quantité totale située dans la plage allant de 0,001 à 3 % en poids, par rapport au poids total d'une composition contenant les oligopeptides, éventuellement un support et éventuellement d'autres ingrédients,
b) application locale des oligopeptides fournis à l'étape de procédé a) sur les cheveux et les follicules capillaires,
c) pose des oligopeptides appliqués sur les cheveux et les follicules capillaires à l'étape b) pendant une période située dans la plage allant de 30 secondes à 2 heures,
d) éventuellement, rinçage de l'agent cosmétique laissé à poser sur les cheveux et les follicules capillaires à l'étape de procédé c).
